(19) **Europäisches Patentamt / European Patent Office / Office européen des brevets**

(11) **EP 2 380 104 B1**

(12) **EUROPEAN PATENT SPECIFICATION**

(45) Date of publication and mention
of the grant of the patent:
**27.05.2020 Bulletin 2020/22**

(21) Application number: **09801257.8**

(22) Date of filing: **19.11.2009**

(51) Int Cl.:
***G16H 10/60*** (2018.01)    ***G16H 10/00*** (2018.01)

(86) International application number:
**PCT/IB2009/055188**

(87) International publication number:
**WO 2010/070488 (24.06.2010 Gazette 2010/25)**

(54) **DISTRIBUTED PATIENT REGISTRIES FOR FEDERATED PACS**

VERTEILTE PATIENTENREGISTRIERDATENBANKEN FÜR FÖDERIERTE PACS

REGISTRES DE PATIENTS DISTRIBUÉS POUR SYSTÈME FÉDÉRÉ D'ARCHIVAGE ET DE TRANSMISSION D'IMAGES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.12.2008 US 138319 P**

(43) Date of publication of application:
**26.10.2011 Bulletin 2011/43**

(73) Proprietor: **Koninklijke Philips N.V.**
**5656 AG Eindhoven (NL)**

(72) Inventors:
• **VDOVJAK, Richard**
 **NL-5621 BA Eindhoven (NL)**

• **BUCUR, Anca, Ioana, Daniela**
 **NL-5621 BA Eindhoven (NL)**
• **REUZEL, Johan, Gerhard, Herman**
 **NL-5621 BA Eindhoven (NL)**

(74) Representative: **Beck, Christopher Andrew et al**
**Philips International B.V.**
**Intellectual Property & Standards**
**High Tech Campus 5**
**5656 AE Eindhoven (NL)**

(56) References cited:
**WO-A1-2007/089514    US-A1- 2002 103 811**
**US-A1- 2003 088 438    US-A1- 2005 246 205**
**US-A1- 2007 078 856**

## Description

### Background

Some of the largest hospitals in the United States are federated health care organizations comprising many autonomous hospital sites. Each autonomous hospital site will typically include its own facilities for conducting tests, studies, investigations, procedures, and etc. on patients and storing the results thereof, including patient images. One common system for storing such results is a Picture Archiving Communication System ("PACS").

US patent application published as US 2007/0078856 A1 describes optimizing the time and effort required to locate all data on a given entity that may span multiple data nodes in a distributed environment. For example, which may be used to locate nodes within the distributed environment that store electronic healthcare records. A poll request from a first node to a second node may include electronic records existence data indicating data nodes known to have, or not have, records related to a given individual. This information is used to minimize the number of nodes that need to be polled to arrive at the complete aggregation of patient records that exist within a given set of nodes.

A federated health care organization may wish to integrate the PACS of its various hospital sites in order to provide data sharing across the entire organization or federation.

### Summary of the Invention

The invention is defined by the independent claims. The dependent claims define advantageous embodiments.

Deleted without prejudice.

Deleted without prejudice.

### Brief Description of the Drawings

Figure 1 shows an exemplary system for sharing medical information among locations of a federated health care system.

Figure 2 shows an exemplary method for requesting and retrieving patient information from distributed databases within a federated health care system.

### Detailed Description

The following exemplary embodiments may be further understood with reference to the following description and the appended drawings, wherein like elements are referred to with the same reference numerals. Described are exemplary systems and methods for integrating PACS from various hospital sites in different locations to form a federated PACS system that allows for data sharing among those various hospital sites.

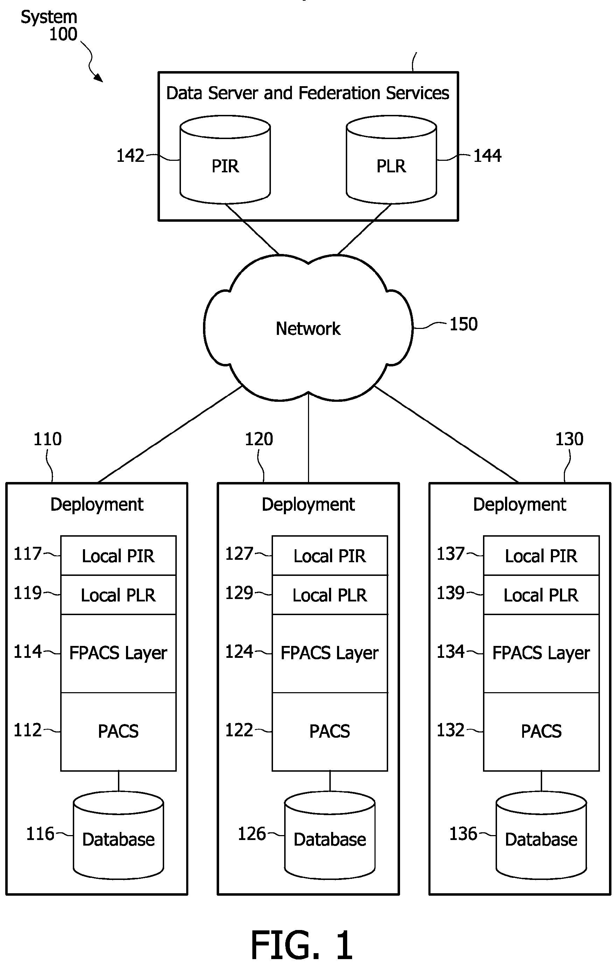

Figure 1 illustrates an exemplary federated PACS ("FPACS") system 100. The system 100 includes local FPACS deployments 110, 120 and 130 and a central FPACS data server and federation services 140 (hereinafter referred to as "data server 140"). The deployments 110, 120 and 130 and the data server 140 communicate by means of a network 150 (e.g., a WAN, the Internet). While Fig. 1 illustrates a system 100 with three local deployments, those having skill in the art will understand that this depiction is only exemplary and that other FPACS implementations may include tens or even hundreds of local deployments. Each of the deployments is typically located at a separate hospital site within a federated health care network, or at different departments within the same hospital. Communication among the local deployments 110, 120 and 130 and the data server 140 is conducted using one or a combination of protocols such as Health Level 7 ("HL7"), Digital Imaging and Communications In Medicine ("DICOM"), and other protocols such as standard or proprietary communication protocols.

The PACS deployments 110, 120 and 130 each include PACS 112, 122 and 132, respectively. The PACS 112, 122 and 132 are typically pre-existing systems used to locally index patient images. Each of the PACS 112, 122 and 132 indexes patient images using a local patient identifier (e.g., social security number, insurance policy number, hospital patient ID, etc.) that may differ among the different PACS 112, 122 and 132. The deployments 110, 120 and 130 also include local FPACS communication layers 114, 124 and 134. The communication layers 114, 124 and 134 route data queries between their corresponding local PACS 112, 122 and 132 and the network 150. The deployments 110, 120 and 130 include image databases 116, 126 and 136 where patient images are stored. The deployments 110, 120 and 130 also include local patient identity registries ("PIR") 117, 127 and 137 (which may be implemented, for example, using cross-referencing ("PIX") databases) and local patient/study location registries ("PLR") 119, 129 and 139, which will be described in greater detail below.

The data server 140 includes a global PIR 142 (which is implemented, for example, by a PIX database) and a global PLR 144. The global PIR 142 integrates the various local patient identifiers used by the PACS 112, 122 and 132 into a global database. The global database defines each patient by a global patient identifier and links the global patient identifiers to the various local patient identifiers. Thus, a local FPACS deployment (e.g., the deployment 110) can query the global PIR 142 using the local patient identifier under which a patient is known by its corresponding PACS (e.g., PACS 112) and retrieve the corresponding global patient identifier for that patient.

The global PLR 144 stores, for each patient, all locations where there are studies for that patient. A study contains one image, a series of images, or several series of images originating in one or several modalities, and metadata associated with the one or more images. Patients are identified in the global PLR 144 by the global patient identifiers as defined in the global PIR 142. The global PLR 144 is initially generated by aggregating relevant metadata from each of the PACS 112, 122 and 132 at the time of generation. It may then be updated by adding a new record to the global PLR 144 when a new patient is registered at one of the PACS 112, 122 and 132; when this occurs, the global PIR 142 will also be queried to determine whether the patient is already known using an existing global patient identifier. When a new patient is introduced at a hospital site, he/she is given a new local patient identifier. To detect whether the patient is already known at other sites and identified by a global patient identifier at the global PIR 142, the new patient is matched by comparing demographic data (e.g., name, address, date of birth, etc.) with the patients registered in the global PIR 142 using existing identity matching techniques. By storing only location information for each patient (as opposed to a centralized data storage system containing patient images), the database can be kept at a manageable size. For example, for a federated health care network with ten locations attending one million patients, the solution can be implemented with a maximum database size of ten million rows, in the extreme scenario where all patients have data at all locations, each row simply storing a local patient identifier and a location where there is data for the patient. As a rough estimate, this database could be implemented to have a maximum size of 50 megabytes.

The global PLR 144 can be modified to store a timestamp of the latest study performed at each institution. Thus, through such a modification, it would be possible for database queries to exclude hospital sites holding studies older than a certain threshold date, which can be predetermined, provided by the user, defined in the system based on the preferences of each institution, etc. A global PLR 144 storing timestamps only adds one extra field per database row (in order to store the timestamp), and thus does not result in a significant increase in database size over a more basic global PLR 144 not modified with timestamp capability. An implementation of the global PLR 144 that stores timestamps can be updated each time a new study is introduced into one of the PACS 112, 122 and 132, or at a regular schedule with a predetermined frequency (daily, weekly, etc.).

The global PLR 144 can also be modified to further store relevant metadata in addition to patient location information. Relevant metadata can be useful because the mere fact that a study is recent does not necessarily make it relevant; for example, a patient seeking care at an orthopedic clinic within a health care network may have entirely irrelevant, though recent, prior studies in an eye clinic. Thus, information from metadata about the nature of a study can be helpful. Relevant metadata may include one or more of a study ID, a body part, a modality and an exam code, or other possibilities not described here. The addition of metadata will result in an increase in the size of the database of the PLR 144, but the size will still be within the manageable size limits of a modern database management system. This type of PLR 144 also allows for the generation of a timeline of relevant prior studies for a patient study stored at one PACS (e.g., PACS 112) without sending queries to the other PACS (e.g., PACS 122 and 132) where those prior studies may reside. Studies can then be retrieved at the user's request. As described above, location tables for this type of global PLR 144 can be updated with each new study or at desired regular intervals (e.g., at night or over weekends in order to take advantage of lighter traffic on the network 150).

The local PIRs 117, 127 and 137 store subsets of the global PIR 142 at the local deployments 110, 120 and 130. The subsets are defined to contain the global patient identifiers and the corresponding local patient identifiers for all patients that also have a local patient identifier at the current location. For example, the local PIR 117 stores the subset of the global PIR 142 selected to include all global and local patient identifiers of all patients that also have a local patient identifier in the PACS 112. In other words, the PIR 117 stores the global patient identifiers and all existing local patient identifiers for all patients that have had studies performed at the location of the deployment 110. This may be represented using the following SQL query, in which PIR represents the PIR 142, PID represents a global and/or local patient identifier, PLR represents the global PLR 144, and PACS represents the local PACS 112:

```
Select * from PIR where PID in (select PID from PLR
              where location = "PACS")
```

The local PLR databases 119, 129 and 139 similarly store subsets of the global PLR 144. At each local deployment, this subset includes the portion of the index containing pointers to locations storing images of patients that have a local patient identifier at the current location, except for those images stored at the same location. For example, the local PLR 119 stores the subset of the global PLR 144 selected to include global patient identifiers and pointers to all locations storing images of patients that have a local patient identifier in the PACS 112, except for those referring to studies contained in the database 116. In other words, the PLR 119 stores pointers to locations storing studies for all patients that have had studies performed at the location of the deployment 110, except for location information concerning those images pertaining to studies performed at the location of the deployment 110 and thus stored locally rather than remotely.

The records stored in the local PLRs 119, 129 and 139 may contain, in addition to global patient identifiers and location information, time stamps of the latest study executed at the referred remote location for the patient, or additional relevant study metadata (e.g., modality, study identifier, exam code, body part, etc.). When relevant metadata is maintained, the local PLR 119, 129 or 139 contains one index (record) for each patient remote study. For systems that include a global PLR 144 that stores either time stamps or full metadata, as described above, the local PLR databases 119, 129 and 139 also store time stamps or full metadata for their appropriate subset of the index. The construction of the local PLR can be represented using the following SQL query, which uses the same designations as above:

```
        Select * from PLR where location<> "PACS" and
   PID in (select PID from PLR where location = "PACS")
```

The local PIRs 117, 127 and 137 and the local PLR databases 119, 129 and 139 are originally established when the global PIR 142 and the global PLR 144 are created to link the various local PACS locations. Subsequently, they are periodically updated with current data. Updates are preferably done infrequently and in bulk rather than more frequently at the time queries are pending. Thus, updates are typically done at times of light network loading (e.g., overnight, on weekends, etc.). Updates may be "pushed" from the global PIR 142 and the global PLR 144 or may be "pulled" by the local deployments. By storing this relevant subset locally, network traffic due to queries can be minimized, and a timeline with relevant prior studies for each study can be built without querying the data server 140. Therefore, this timeline can also be built reliably when the local deployment is disconnected from the network 150 for a short period of time. When the local deployment stays disconnected for longer period, the timeline built based exclusively on local information may become outdated.

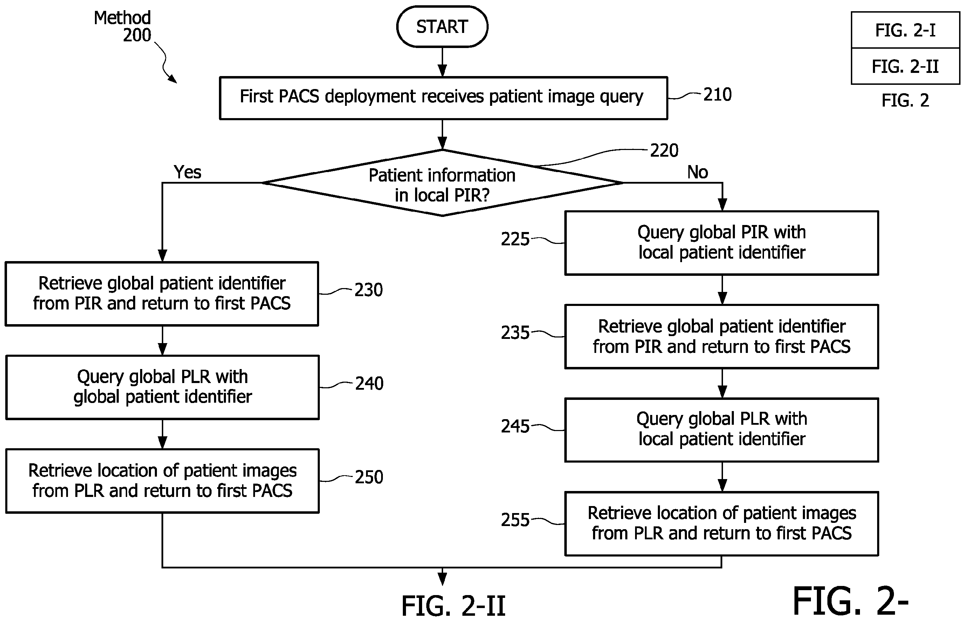

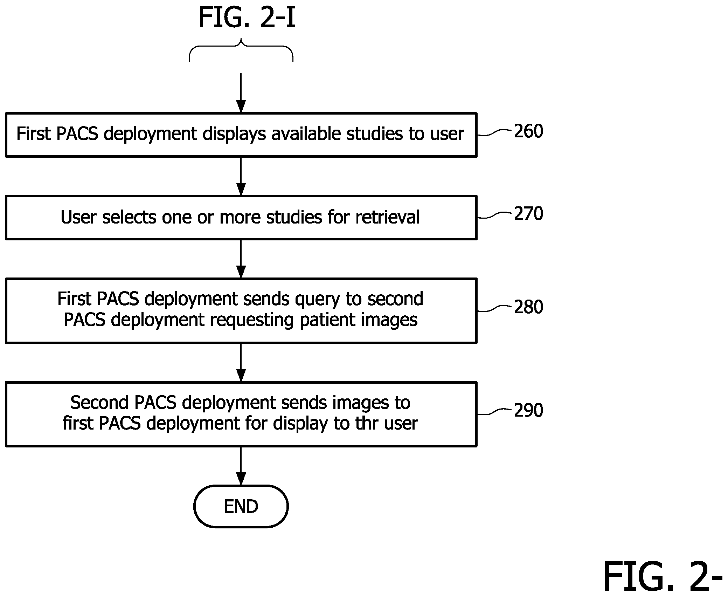

Figure 2 shows an exemplary method 200 for routing a query for patient images. The method 200 is initiated, for example, by a user of one of the local PACS 112, 122 and 132 of Fig. 1; the description herein refers to a query initiated by a user of PACS 112. In step 210, the PACS 112 receives a query for patient information from a user (e.g., a doctor, a nurse, a testing technician, or other type of clinician, etc.). The query received in step 210 identifies the patient with a local patient identifier that is local to the PACS 112, as described above. Depending on the type of global PLR 144 that is implemented with the system 100, the query may also include a timestamp cutoff point (for a global PLR 144 that stores timestamps) or a search criterion corresponding to the nature of the information that the user wishes to retrieve (for a global PLR 144 that stores full metadata).

In step 220, the PACS 112 queries its local PIR 117 to determine whether there is a locally stored global patient identifier corresponding to the local patient identifier used in step 210. As described above, a patient who has previously received treatment at the location of the PACS 112 will be indexed in the local PIR 117 with a global patient identifier and one or more corresponding local patient identifiers; however, a patient who has been treated elsewhere in the federated healthcare network but not at the current location will not be indexed. If the patient is indexed in the local PIR 117, the method proceeds to step 230, wherein the local PIR 117 returns the global patient identifier for the patient to the PACS 112.

Subsequently, in step 240, the PACS 112 generates a query and sends it to a subset of the local PLR 119. This second query identifies the patient by the global patient identifier received in step 230. For a basic implementation of the global PLR 144 and its subsets in local PLRs 119, 129 and 139, solely the global patient identifier is required for this query. Alternately, for a global PLR 144 and its subsets in local PLRs 119, 129 and 139 storing timestamp information, the query would include the global patient identifier and the desired timestamp cutoff submitted by the user in step 210. Similarly, for a global PLR 144 and subsets in local PLRs 119, 129 and 139 storing full metadata information, the query would include the global patient identifier and the search criterion or criteria corresponding to the metadata as selected by the user in step 210.

Next, in step 250, the local PLR 119 retrieves information and returns it to the PACS 112. The information retrieved corresponds to the global patient identifier as retrieved in step 230 and transmitted in step 240, and provides the PACS 112 with all the locations of studies for the patient. For example, the patient may have had studies previously recorded at the hospital sites corresponding to PACS 122 and 132 (i.e., stored in databases 126 and 136). Locations are provided to the PACS 112 in the form of network addresses (e.g., IP addresses, network paths, etc.). In other implementations of the local PLR 119, added functionality is added to this retrieval. In a local PLR 119 implementation with timestamp records, only locations storing studies more recent than a certain threshold may be provided in response to the query; in a local PLR 119 storing full metadata records, only locations of studies relevant to the search terms may be provided. For example, assume that the patient whose records are currently being searched at the location of the PACS 112 was treated for a broken leg two years ago at the location of PACS deployment 120 and for glaucoma four years ago at the location of PACS deployment 130. A local PLR 19 that supports timestamp searching may return the location of the study in PACS 122 (i.e., in database 126) if the search has specified a cut-off point of three years. However, if the patient is seeking treatment for an eye condition, a local PLR 119 that stores all relevant metadata may be searched with a query that returns the location of the study in PACS 132 (i.e., in database 136), though it is less recent. Those having skill in the art will understand that a local PLR 119 that stores all metadata may also support the ability to search by timestamp or by any of the other stored metadata.

If, in step 220, it is determined that the patient is not indexed in the local PIR 117 (i.e., the patient has not previously been known in the local system, so the global patient identifier, if any, is not known), the method proceeds to step 225, where a query is sent to the global PIR 142 requesting the same information. The query is sent from the PACS 112 to its corresponding FPACS communication layer 114, via the network 150, to the GLOBAL PIR 142. As described above, one or more protocols such as HL7, DICOM, and other standard or proprietary protocols may be used. In step 235, the global PIR 142 retrieves the global patient identifier, corresponding to the local patient identifier used in step 210, and returns it to the PACS 112 in the same manner as step 225.

Next, in step 245, the PACS 112 generates a query, similar to that sent to the local PLR 119 in step 240, and sends it to the global PLR 144. As described above for step 235, transmission is accomplished via the FPACS communication layer 114 and the network 150. In step 255, the global PLR 144 retrieves data in response to the query of step 245 and returns it to the PACS 112 via the network 150 and the FPACS layer communication 114. Data retrieved in step 255 will be similar to that retrieved in step 250, discussed above. After step 255 is completed, the method continues with step 260.

In step 260, the results of the query sent in step 240 or step 245 are provided to the user of the PACS 112 (e.g., in a timeline). For a basic global PLR 144 and subsets in PLRS 119, 129 and 139, the results are simply a list of locations (e.g., for the example described above, the user would be informed that the patient has one previous study in the database 126 and one in the database 136). For a timestamp global PLR 144 and subsets in PLRs 119, 129 and 139, the list would be provided with locations and corresponding timestamps (e.g., for the example described above, the user would be informed that the patient has a previous study stored in database 126, together with the date that study occurred; as described above, the study stored in database 136 would not be returned because it is beyond the specified time threshold). For a full metadata global PLR 144 and subsets in PLRS 119, 129 and 139, the provided list would include locations, timestamps, and any other metadata corresponding to the retrieved records (e.g., for the example described above, the user would be informed of the prior treatment for glaucoma and its corresponding images stored at the location of PACS deployment 132; the prior study undertaken at the location of PACS 122 would not be returned because it is not relevant to the search the user is performing.)

In step 270, the user of PACS 112 selects one or more studies from those provided in step 260 for retrieval. In another exemplary embodiment, all relevant studies may be prefetched for the user's viewing. This selection may be accomplished by selecting studies from a list or timeline (e.g., with a mouse), selecting a "retrieve all" command, or any other process known in the art. In step 280, the request is sent by the FPACS communication layer 114, via the network 150, to the location where images are stored. For example, if the images to be retrieved are located in database 126, the request would be passed from FPACS communication layer 114, through the network 150, to the FPACS layer 124, the PACS 122, and the database 126. This request is not transmitted to or through data server 140, as the location information has already been extracted. In step 290, the requested images are transmitted from their storage location (e.g., database 126) to the requesting user, via the same data path, and displayed to the user. Those of skill in the art will understand that display to the user may include the option to print images, etc. Following step 290, the method 200 terminates. Those of skill in the art will understand that the method 200 may terminate prior to this step if, at any point, no results are returned in response to a database query.

By storing studies locally at various PACS sites while storing indexes at a central location, a very efficient data sharing system can be provided. This type of system is scalable to large systems including tens or hundreds of hospitals and allows a physician at any participating hospital to retrieve images located at any other hospital within the system. Further, by storing subsets of the indexes locally at each of the PACS sites, network usage may be minimized at peak times when queries are typically being sent.

**Claims**

1. A system for sharing medical information among a plurality of locations, comprising:

a plurality of local Picture Archiving Communication Systems, PACS, (112, 122, 132) storing patient studies, each patient study being indexed by a local patient identifier;

a plurality of local patient identity registries, PIRs, (117, 127, 137) each adapted to store a local identity list including a respective global patient identifier and corresponding to local patient identifier for each of a plurality of patients having studies stored on a corresponding one of the local PACS (112, 122, 132)

a plurality of local location registries, PLRs (119, 129, 139) reach adapted to store a local index of the patient studies stored on the local PACS (112, 122, 132) for each of the plurality of patients, the index including a storage location of each study and the corresponding global patient identifier;

wherein the plurality of local PACS (112, 122, 132), the plurality of local PIRs (117, 127, 137) and the plurality of local PLRs (119, 129, 139) are arranged in a plurality of local PACS deployments (110, 120, 130), each local PACS deployment (110, 120, 130) comprising a respective local PACS (112, 122, 132), a respective local PIR (117, 127, 137) and a respective local PLR (119, 129, 139);

a global PIR (142), located remotely from the local PACS (112, 122, 132), the local PIRs (117, 127, 137) and the local PLRs (119, 129, 139), adapted to store a global identity list including a global patient identifier for each patient having studies stored in the local PACS (112, 122, 132), the global patient identifier being linked with corresponding local patient identifiers; and

a global PLR (144), located remotely from the local PACS (112, 122, 132), the local PIRs (117, 127, 137) and the local PLR (119, 129, 139), adapted to store a global index of the patient studies stored on the local PACS (112, 122, 132) for each patient, the index including a storage location of each study and the corresponding global patient identifier.

2. The system of claim 1, wherein the local identity list is a subset of the global identity list.

3. The system of claim 1, wherein the local index is a subset of a global index.

4. The system of claim 1, wherein one of the local PACS (112, 122, 132) is adapted to send a query including one of the local patient identifiers to a corresponding one of the local PIRs (117, 127, 137) which is adapted to determine if local identity list includes the one of the local patient identifiers and return a corresponding one of the global patient identifiers to the local PACS (112, 122, 132) if the local patient identifier is included in the local identity list.

5. The system of claim 4, wherein the one of the local PACS (112, 122, 132) is adapted to send a further query including the returned one of the global patient identifiers to a corresponding one of the local PLRs (119, 129, 139) which is adapted to return a listing of patient studies of the patient identified by the returned one of the global patient identifiers, the listing including the storage location of each patient study.

6. The system of claim 4, wherein, the one of the local PACS (112, 122, 132) is adapted to, if the query includes a local patient identifier not included in the local identity list, redirect the query to the global PIR (142) which is adapted to return a corresponding one of the global patient identifiers.

7. The system of claim 6, wherein the one of the local PACS (112, 122, 132) is adapted to send a further query including the returned one of the global patient identifiers to the global PLR (144) which is adapted to return the storage location of each patient study.

8. A computer implemented method for routing a query in a system according to claim 1, the method in a PACS (112, 122, 132) of a first local PACS deployment (110, 120, 130) comprising:

receiving (210), at the PACS (112, 122, 132), from a user of the first local PACS deployment (110, 120, 130), a first query for patient information, the first query including a local patient identifier;

determining (220) whether the local identity list stored in the local PIR (117, 127, 137) in the first local PACS deployment (110, 120, 130) includes the local patient identifier;

receiving (230), from the local PIR (117, 127, 137) in the first local PACS deployment (110, 120, 130), when the local identity list includes the local patient identifier, a global patient identifier stored in the local identity list corresponding to the local patient identifier; and

sending (225), via a network (150), when the local identity list does not include the local patient identifier, the first query to the global PIR (142) storing the global identity list remotely from the local identity list, wherein the local identity list is a subset of the global identity list.

9. The method of claim 8, further comprising:

receiving (235) from the global identity list stored in the global PIR (142) a global patient identifier stored in the global identity list corresponding to the local patient identifier.

10. The method of claim 8, further comprising:

sending (240) a second query including the one of the global patient identifiers received from the local identity list to a local index stored in the local PLR (119, 129, 139) in the first local PACS deployment (110, 120, 130), wherein the local index is a subset of the global index stored remotely from the local index; and
receiving (250) from the local index a listing of patient studies corresponding to the one of the global patient identifiers, the listing including a storage location of each patient study.

11. The method of claim 9, further comprising:

sending (245) a second query including the one of the global patient identifiers received from the global identity list to the global PLR (144) storing the global index; and
receiving (255) from the global index a listing of patient studies corresponding to the one of the global patient identifiers, the listing including a storage location of each patient study.

**Patentansprüche**

1. System zum Austausch medizinischer Informationen zwischen mehreren Orten, umfassend:

eine Vielzahl von lokalen Bildarchivierungs-Kommunikationssystemen, PACS, (112, 122, 132), die Patientenstudien speichern, wobei jede Patientenstudie durch eine lokale Patientenkennung indiziert wird;
eine Vielzahl von lokalen Patientenidentitätsregistern, PIR (117, 127, 137), die jeweils angepasst sind, um eine lokale Identitätsliste zu speichern, die eine jeweilige globale Patientenkennung enthält und der lokalen Patientenkennung für jeden einer Vielzahl von Patienten entspricht, deren Studien auf einem entsprechenden der lokalen PACS (112, 122, 132) gespeichert wurden haben;
eine Vielzahl von lokalen Standortregistern, PLR (119, 129, 139), die jeweils angepasst sind, um einen lokalen Index der auf dem lokalen PACS gespeicherten Patientenstudien (112, 122, 132) zu speichern, für jeden der Vielzahl von Patienten, der Index enthaltend einen Speicherort jeder Studie und die entsprechende globale Patientenkennung;
wobei die Vielzahl lokaler PACS (112, 122, 132), die Vielzahl lokaler PIR (117, 127, 137) und die Vielzahl lokaler PLR (119, 129, 139) in mehreren lokalen PACS-Bereitstellungen (110, 120, 130) angeordnet sind, wobei jede lokale PACS-Bereitstellung (110, 120, 130) eine jeweilige lokale PACS (112, 122, 132), ein jeweiliges lokales PIR (117, 127, 137) und ein jeweiliges lokales PLR (119, 129, 139) umfasst;
ein globales PIR (142), das sich entfernt von der lokalen PACS (112, 122, 132), der lokalen PIR (117, 127, 137) und der lokalen PLR (119, 129, 139) befindet, das angepasst ist, um einer globalen Identitätsliste zu speichern, die einer globale Patientenkennung für jeden Patienten enthält, dessen Studien in dem lokalen PACS (112, 122, 132) gespeichert sind, wobei die globale Patientenkennung mit entsprechenden lokalen Patientenkennungen verknüpft ist; und
ein globales PLR (144), das sich entfernt von der lokalen PACS (112, 122, 132), der lokalen PIR (117, 127, 137) und der lokalen PLR (119, 129, 139) befindet, das angepasst ist, um einen globalen Index der auf dem lokalen PACS (112, 122, 132) gespeicherten Patientenstudien für jeden Patienten zu speichern, wobei der Index einen Speicherort jeder Studie und die entsprechende globale Patientenkennung enthält.

2. System nach Anspruch 1, wobei die lokale Identitätsliste eine Teilmenge der globalen Identitätsliste ist.

3. System nach Anspruch 1, wobei der lokale Index eine Teilmenge eines globalen Index ist.

4. System nach Anspruch 1, wobei eines der lokalen PACS (112, 122, 132) angepasst ist, um eine Abfrage einschließlich einer der lokalen Patientenkennungen an ein entsprechendes der lokalen PIR (117, 127, 137) zu senden, das angepasst ist, um zu bestimmen, ob die lokale Identitätsliste die eine der lokalen Patientenkennungen enthält, und eine entsprechende der globalen Patientenkennungen an das lokale PACS (112, 122, 132) zurückzugeben, wenn die lokale Patientenkennung in der lokalen Identitätsliste enthalten ist.

5. System nach Anspruch 4, wobei dasjenige der lokalen PACS (112, 122, 132) angepasst ist, um eine weitere Abfrage einschließlich der zurückgegebenen der globalen Patientenkennungen an eine entsprechende der lokalen PLR (119, 129, 139) zu senden, die angepasst ist, um eine Liste von Patientenstudien des Patienten zurückzugeben, der durch die zurückgegebene der globalen Patientenkennung identifiziert wurde, wobei die Liste den Speicherort jeder Patientstudie enthält.

6. System nach Anspruch 4, wobei dasjenige der lokalen PACS (112, 122, 132) angepasst ist, wenn die Abfrage eine lokale Patientenkennung enthält, die nicht in der lokalen Identitätsliste vorhanden ist, um die Abfrage an die globale PIR umzuleiten (142), das angepasst ist, um eine entsprechende der globalen Patientenkennungen zurückzugeben.

7. System nach Anspruch 6, wobei dasjenige der lokalen PACS (112, 122, 132) angepasst ist, um eine weitere Abfrage einschließlich der zurückgegebenen der globalen Patientenkennungen an das globale PLR (144) zu senden, das angepasst ist, um den Speicherort jeder Patientstudie zurückzugeben.

8. Computerimplementiertes Verfahren zum Weiterleiten einer Abfrage in einem System gemäß Anspruch 1, wobei das Verfahren in einem PACS (112, 122, 132) einer ersten lokalen PACS-Bereitstellung (110, 120, 130) umfasst:

> Empfangen (210) bei dem PACS (112, 122, 132) von einem Benutzer der ersten lokalen PACS-Bereitstellung (110, 120, 130) eine erste Abfrage nach Patienteninformationen, wobei die erste Abfrage eine lokale Patientenkennung enthält;
> Bestimmen (220), ob die lokale Identitätsliste, die in dem lokalen PIR (117, 127, 137) in der ersten lokalen PACS-Bereitstellung (110, 120, 130) gespeichert ist, die lokale Patientenkennung enthält;
> Empfangen (230) von dem lokalen PIR (117, 127, 137) in der ersten lokalen PACS-Bereitstellung (110, 120, 130), wenn die lokale Identitätsliste die lokale Patientenkennung enthält, eine globale Patientenkennung, die in der lokalen Identitätsliste gespeichert ist, die der lokalen Patientenkennung entspricht; und
> Senden (225) über ein Netzwerk (150), wenn die lokale Identitätsliste nicht die lokale Patientenkennung enthält, die erste Abfrage an das globale PIR (142), die die globale Identitätsliste fern von der lokalen Identitätsliste speichert, wobei die lokale Identitätsliste eine Teilmenge der globalen Identitätsliste ist.

9. Verfahren nach Anspruch 8, ferner umfassend:
Empfangen (235) von der in dem globalen PIR (142) gespeicherten globalen Identitätsliste eine globale Patientenkennung, die in der globalen Identitätsliste gespeichert ist, die der lokalen Patientenkennung entspricht.

10. Verfahren nach Anspruch 8, ferner umfassend:

> Senden (240) einer zweiten Abfrage, die diejenige der globalen Patientenkennungen enthält, die von der lokalen Identitätsliste empfangen wurden, an einen lokalen Index, der im lokalen PLR (119, 129, 139) in der ersten lokalen PACS-Bereitstellung (110, 120, 130) gespeichert ist, wobei der lokale Index eine Teilmenge des globalen Index ist, der remote vom lokalen Index gespeichert ist, wobei der lokale Index eine Teilmenge des globalen Index ist, der fern vom lokalen Index gespeichert wird; und
> Empfangen (250) einer Liste von Patientenstudien aus dem lokalen Index, die diejenigen der globalen Patientenkennungen entsprechen, wobei die Liste einen Speicherort jeder Patientstudie enthält.

11. Verfahren nach Anspruch 9, ferner umfassend:

> Senden (245) einer zweiten Abfrage, die diejenige der globalen Patientenkennungen enthält, die von der globalen Identitätsliste empfangen wurden, an das globale PLR (144), das den globalen Index speichert; und
> Empfangen (255) einer Liste von Patientenstudien aus dem globalen Index, die diejenigen der globalen Patientenkennungen entsprechen, wobei die Liste einen Speicherort jeder Patientstudie enthält.

**Revendications**

1. Système pour partager des informations médicales parmi une pluralité d'emplacements, comprenant:

> une pluralité de systèmes d'archivage et de transmission d'images locaux, PACS, (112, 122, 132) stockant des études de patients, chaque étude de patient étant indexée par un identifiant de patient local;
> une pluralité de registres d'identités de patients locaux, PIR, (117, 127, 137) chacun adapté pour stocker une liste d'identités locale comprenant un identifiant de patient global respectif et correspondant à l'identifiant de patient local pour chacun d'une pluralité de patients ayant des études stockées sur un correspondant des PACS locaux (112, 122, 132);

une pluralité de registres d'emplacements locaux, PLR (119, 129, 139) chacun adapté pour stocker un index local des études de patients stockées sur les PACS locaux (112, 122, 132) pour chacun de la pluralité de patients, l'index comprenant un emplacement de stockage de chaque étude et l'identifiant du patient global correspondant;

où la pluralité de PACS locaux (112, 122, 132), la pluralité de PIR locaux (117, 127, 137) et la pluralité de PLR locaux (119, 129, 139) sont disposés dans une pluralité de déploiements de PACS locaux (110, 120, 130), chaque déploiement de PACS local (110, 120, 130) comprenant un PACS local respectif (112, 122, 132), un PIR local respectif (117, 127, 137) et un PLR local respectif (119, 129, 139);

un PIR global (142), situé à distance des PACS locaux (112, 122, 132), des PIR locaux (117, 127, 137) et des PLR locaux (119, 129, 139), adapté pour stocker une liste d'identités globale incluant un identifiant de patient global pour chaque patient ayant des études stockées dans les PACS locaux (112, 122, 132), l'identifiant du patient global étant lié aux identifiants de patients locaux correspondants; et

un PLR global (144), situé à distance des PACS locaux (112, 122, 132), des PIR locaux (117, 127, 137) et des PLR locaux (119, 129, 139), adapté pour stocker un index global des études de patients stockées sur les PACS locaux (112, 122, 132) pour chaque patient, l'index comprenant un emplacement de stockage de chaque étude et l'identifiant du patient global correspondant.

2. Système selon la revendication 1, dans lequel la liste d'identités locale est un sous-ensemble de la liste d'identités globale.

3. Système selon la revendication 1, dans lequel l'index local est un sous-ensemble d'un index global.

4. Système selon la revendication 1, dans lequel un des PACS locaux (112, 122, 132) est adapté pour envoyer une requête comprenant un des identifiants des patients locaux à un correspondant des PIR locaux (117, 127, 137) qui est adapté pour déterminer si la liste d'identités locale comprend un des identifiants de patients locaux et renvoyer un correspondant des identifiants de patients globaux aux PACS locaux (112, 122, 132) si l'identifiant de patient local est inclus dans la liste d'identités locale.

5. Système selon la revendication 4, dans lequel ce l'un des PACS locaux (112, 122, 132) est adapté pour envoyer une autre requête incluant celui retourné des identifiants de patients globaux à un correspondant des PLR locaux (119, 129, 139) qui est adapté pour renvoyer une liste des études de patients du patient identifié par celui renvoyé des identifiants de patients globaux, la liste comprenant l'emplacement de stockage de chaque étude de patient.

6. Système selon la revendication 4, dans lequel ce l'un des PACS locaux (112, 122, 132) est adapté pour, si la requête comprend un identifiant de patient local non inclus dans la liste d'identités locale, rediriger la requête vers le PIR global (142) qui est adapté pour renvoyer un correspondant des identifiants de patients globaux.

7. Système selon la revendication 6, dans lequel ce l'un des PACS locaux (112, 122, 132) est adapté pour envoyer une autre requête incluant celui renvoyé des identifiants de patients globaux au PLR global (144) qui est adapté pour renvoyer l'emplacement de stockage de chaque étude de patient.

8. Procédé implémenté par ordinateur pour acheminer une requête dans un système selon la revendication 1, le procédé dans un PACS (112, 122, 132) d'un premier déploiement de PACS local (110, 120, 130) comprenant:

recevoir (210), aux PACS (112, 122, 132), d'un utilisateur du premier déploiement de PACS local (110, 120, 130), une première requête d'informations du patient, la première requête incluant un identifiant de patient local;
déterminer (220) si la liste d'identités locale stockée dans le PIR local (117, 127, 137) dans le premier déploiement de PACS local (110, 120, 130) inclut l'identifiant du patient local;
recevoir (230), du PIR local (117, 127, 137) dans le premier déploiement de PACS local (110, 120, 130), lorsque la liste d'identités locale comprend l'identifiant du patient local, un identifiant de patient global stocké dans la liste d'identités locale correspondante à l'identifiant du patient local; et
envoyer (225), via un réseau (150), lorsque la liste d'identités locale ne comprend pas l'identifiant du patient local, la première requête au PIR global (142) stockant la liste d'identités globale à distance à partir de la liste d'identités locale, où la liste d'identités locale est un sous-ensemble de la liste d'identités globale.

9. Procédé selon la revendication 8, comprenant en outre:

recevoir (235) de la liste d'identités globale stockée dans le PIR global (142) un identifiant de patient global stocké dans la liste d'identités globale correspondant à l'identifiant du patient local.

10. Procédé selon la revendication 8, comprenant en outre:

envoyer (240) une deuxième requête comprenant celui des identifiants de patients globaux reçu de la liste d'identités locale à un index local stocké dans le PLR local (119, 129, 139) dans le premier déploiement de PACS local (110, 120, 130), où l'index local est un sous-ensemble de l'index global stocké à distance à partir de l'index local; et
recevoir (250) de l'index local une liste d'études de patients correspondant à celle des identifiants de patients globaux, la liste comprenant un emplacement de stockage de chaque étude de patient.

11. Procédé selon la revendication 9, comprenant en outre:

envoyer (245) une seconde requête comprenant celui des identifiants de patients globaux reçu de la liste d'identités globale au PLR global (144) stockant l'index global; et
recevoir (255) de l'index global une liste d'études de patients correspondant à l'un des identifiants de patients globaux, la liste comprenant un emplacement de stockage de chaque étude de patient.

System
100

FIG. 1

FIG. 2-I

FIG. 2-II

FIG. 2

Method 200

START

First PACS deployment receives patient image query — 210

Patient information in local PIR? — 220

Yes

No

Retrieve global patient identifier from PIR and return to first PACS — 230

Query global PLR with global patient identifier — 240

Retrieve location of patient images from PLR and return to first PACS — 250

Query global PIR with local patient identifier — 225

Retrieve global patient identifier from PIR and return to first PACS — 235

Query global PLR with local patient identifier — 245

Retrieve location of patient images from PLR and return to first PACS — 255

FIG. 2-II

FIG. 2-

# FIG. 2-I

First PACS deployment displays available studies to user — 260

User selects one or more studies for retrieval — 270

First PACS deployment sends query to second PACS deployment requesting patient images — 280

Second PACS deployment sends images to first PACS deployment for display to thr user — 290

END

# FIG. 2-

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070078856 A1